# EUROPÄISCHE PATENTSCHRIFT

(11) **EP 0 869 743 B1**
(45) Veröffentlichungstag und Bekanntmachung des Hinweises auf die Patenterteilung: **31.03.2004**
(21) Anmeldenummer: 96943904.1
(22) Anmeldetag: 09.12.1996
(51) Int. Cl.: A61B 18/12

(54) **VORRICHTUNG ZUM VERSCHLIESSEN VON IN DEN UTERUS FÜHRENDEN TUBEN**
DEVICE FOR CLOSING OFF TUBES LEADING INTO THE UTERUS
DISPOSITIF POUR FERMER DES TUBES CONDUISANT DANS L'UTERUS

(30) Priorität: 14.12.1995 DE 19546761
(43) Veröffentlichungstag der Anmeldung: 14.10.1998
(73) Patentinhaber: Starflinger, Franz, 84547 Emmerting (DE)
(72) Erfinder: STARFLINGER, Franz, D-84547 Emmerting (DE); SCHMIDINGER, Frank, D-84513 Töging (DE)
(74) Vertreter: Finsterwald, Manfred, Dipl.-Ing., Dipl.-Wirtsch.-Ing.
(86) Internationale Anmeldenummer: PCT/EP1996/005500
(87) Internationale Veröffentlichungsnummer: WO 1997/021388

(56) Entgegenhaltungen:
- EP-A- 0 596 436
- WO-A-81/00200
- WO-A-95/34259
- DE-A- 2 913 036
- US-A- 4 011 872

## Beschreibung

Die Erfindung betrifft eine Vorrichtung zum Verschließen von in den Uterus führenden Tuben mit einem bipolaren, mit einer hochfrequenten Stromquelle verbindbaren Koagulationssystem, wobei am Ende einer rohrförmigen Träger- und/oder Inspektionseinheit ein Funktionskopf mit zumindest zwei in Form von Klemmelementen ausgebildeten Elektroden vorgesehen ist, die zwischen einer passiven Position und einer aktiven Koagulationsposition verstellbar sind.

Es ist bekannt und üblich, die Sterilisation bei der Frau mittels Bauchspiegelung und Elektrokoagulation der Eileiter mittels bipolarer Koagulationszangen auf dem transabdominalen Weg durchzuführen. Dabei werden die Tuben bzw. Eileiter mittels der Koagulationszange von außen ergriffen und durch die Einwirkung des zuschaltbaren hochfrequenten Koagulationsstromes verschlossen.

Eine dazu geeignete Vorrichtung ist aus der WO 81/00200 bekannt. Dabei sind die als Klemmelemente ausgebildeten Elektroden an ihren freien Enden hakenförmig gestaltet, um Tuben von außen sicher ergreifen und umfassen zu können. In der passiven Position befinden sich die Elektroden mit Abstand beiderseits der jeweiligen Tube und in der Koagulationsposition wird die Tube von den Elektroden zangenartig umschlossen und zusammengedrückt, so dass dann der Koagulationsvorgang durchgeführt werden kann.

Diese Vorgehensweise ermöglicht zwar die Durchführung von Sterilisationen mit der erforderlichen Sicherheit, aber es müssen dabei auch erhebliche Risikofaktoren in Kauf genommen werden, die durch eventuelle Verwachsungen im Bauch bzw. durch Irritationen des Darms bedingt sein können. Schließlich ist auch zu erwähnen, dass die Durchführung derartiger Sterilisationen meistens stationär im Krankenhaus erfolgt und damit auch entsprechende Kosten entstehen.

Es wurden auch bereits Versuche unternommen, zur Erzielung einer Sterilisation die Eileiterostien, d.h. die Einmündungen der Eileiter in die Gebärmutter mittels chemischer Mittel zu verschließen. Dabei wurde die Möglichkeit genutzt, den Uterus mittels der Methode der Hysteroskopie durch den Muttermund zu inspizieren und damit Zugang zu den Eileiterostien zu erhalten. Es hat sich jedoch gezeigt, dass diese Vorgehensweise nicht zu der geforderten Sicherheit, d.h. nicht zur Gewährleistung der sicheren Sterilisation geeignet ist.

Aufgabe der vorliegenden Erfindung ist es, eine Vorrichtung der eingangs angegebenen Art so auszugestalten, dass mittels eines einfach aufgebauten und problemfrei zu bedienenden Geräts ein sicheres Verschließen der Tuben ermöglicht wird, und zwar unter Vermeidung der mit der transabdominalen Methode verbundenen Risiken. Die Vorrichtung soll es insbesondere ermöglichen, Sterilisationen der angegebenen Art ambulant durchzuführen.

Gelöst wird diese Aufgabe nach der Erfindung im wesentlichen dadurch, dass die Träger- und/oder Inspektionseinheit mit dem Funktionskopf als in den Uterus einführbare Einheit ausgebildet ist und die Enden der Elektroden die Form von Stechspitzen besitzen, die in der passiven Position einen ein Einstechen beiderseits der Eileiteröffnung gewährleistenden gegenseitigen Abstand besitzen, und dass dieser Abstand in der aktiven Koagulationsposition durch eine zur Wirkung kommende Klemmkraft verringert ist.

Mittels der Vorrichtung nach der Erfindung ist es möglich, die Eileitereinmündungen von der Uterushöhle aus zu koagulieren bzw. dauerhaft zu verschließen. Dies ermöglicht vor allem die Ausgestaltung des Funktionskopfes der Vorrichtung, der nach Einführung in die Uterushöhle, die gleichzeitig inspiziert werden kann, so positionierbar ist, dass die beiden Elektroden beiderseits der im Durchmesser circa einen Millimeter messenden Eileiteröffnung eingestochen werden können, so dass sie dann beiderseits des Eileiterkanals in der Stechstellung positioniert sind. Durch Aufbringen einer die Elektroden einander annähernden Klemmkraft und das Zuschalten des Koagulationsstromes wird dann nach der bewährten Koagulationsmethode der jeweiligen Eileiter sicher verschlossen.

Unter Einsparungseffekten von besonderer Bedeutung ist, daß die Vorrichtung nach der Erfindung jederzeit ambulant im Rahmen einer Kurznarkose eingesetzt werden kann und damit den Krankenkassenträgern erhebliche Aufwendungen für sonst erforderliche längere Krankenhausaufenthalte erspart bleiben. Außerdem wird durch die praktische Beseitigung der sich bei Durchführung einer Sterilisation mittels Bauchspiegelung ergebenden Risiken auch dazu beigetragen, daß keine komplikationsbedingt verlängerten Krankenhausaufenthalte notwendig werden.

Besonders vorteilhafte Ausgestaltungen der Erfindung sind in den Unteransprüchen angegeben.

Demgemäß sind die Elektrodenenden in Form feiner Spitzen ausgebildet und mit Schneidkanten versehen, so daß nach der Positionierung der Elektroden beiderseits der Eileiteröffnung ein problemloses Einstechen erfolgen kann und die Elektroden nahezu widerstandsfrei etwa 1 cm in das Gewebe beiderseits des Eileiterkanals hochgeführt werden können.

Die Elektroden können in der in der minimalinvasiven Chirurgie üblichen Weise in einem Führungsrohr angeordnet sein und liegen dann in diesem Führungsrohr dicht aneinander. Werden sie aus dem Führungsrohr durch entsprechende Betätigung ausgefahren, so gelangen sie in eine gegenseitige Distanzposition, aus der sie in die Stechposition überführt werden können.

Besonders vorteilhaft ist die Ausführungsform der Erfindung mit Elektroden, die mit Bimetallelementen zusammenwirken oder aus Bimetallelementen bestehen, weil diese Elektroden nicht notwendigerweise in einem Führungsrohr angeordnet sein müssen, sondern stationär angebracht sein können und sich dabei bereits in der Einstechposition befinden. Auf diese Weise kann eine Schiebemechanik vermieden werden.

Wesentlich für die Erfindung ist, daß zur Überführung der Elektroden von der Stechposition in die Klemm- und Koagulationsposition eine mechanische und / oder elektrische Anordnung zur Klemmkrafterzeugung vorgesehen ist, denn diese Klemmkrafterzeugung ist zur Komprimierung des Ostiums notwendig und Voraussetzung dafür, daß der jeweilige Eileiter durch Koagulation sicher verschlossen werden kann.

Eine mögliche Ausführungsform einer Einrichtung zur mechanischen Klemmkrafterzeugung umfaßt ein relativ zum Führungsrohr verschiebbares, an den Elektroden angreifendes und deren gegenseitigen Abstand verringerndes Betätigungsorgan, das vom außenliegenden Ende der Träger- und / oder Inspektionseinheit her gehandhabt werden kann.

Das Betätigungsorgan besteht zweckmäßigerweise aus einem Hülsenelement aus elektrisch isolierendem Material.

Besonders vorteilhaft unter dem Aspekt der einfachen Bedienbarkeit der Vorrichtung ist es, eine elektrische Anordnung zur Klemmkrafterzeugung zu verwenden, welche mit den Elektroden verbundene Bimetallelemente aufweist, die über zumindest eine zugeordnete Heizwendel unter gleichzeitiger Bewirkung einer gegenseitigen Annäherung der Elektroden aufheizbar sind. In diesem Zusammenhang wiederum von besonderem Vorteil ist es, die Elektroden selbst in Form von Bimetallelementen auszubilden.

Da Bimetallelemente ein ganz definiertes Bewegungsverhalten bei entsprechender Erwärmung besitzen, wird auch ein definiertes Komprimieren des Ostiums sichergestellt.

Die Heizwendeln werden bevorzugt mit einem vom Koagulationsstrom abgezweigten Teilstrom gespeist, so daß auf diese Weise die Bedienung der Vorrichtung weiter wesentlich vereinfacht wird, denn die Zuschaltung des Koagulationsstroms bewirkt ohne weitere sonstige Betätigung die erforderliche Klemmkrafterzeugung. Die träge mechanische Reaktion der Bimetallteile nach Zuschaltung des Heizstroms ist für die Funktionsweise der Vorrichtung von Vorteil. Mittels eines elektrischen Verzögerungsgliedes kann aber gegebenenfalls auch dafür Sorge getragen werden, daß die Zuschaltung des Koagulationsstromes bezüglich der Zuschaltung des Heizwendelstromes zeitlich verzögert wird.

Um vor dem Beginn des Einstechvorgangs eine exakte Positionierung der Elektroden bezüglich der jeweiligen Tubenöffnung zu erleichtern, kann im Führungsrohr ein mittig zwischen den Elektroden verfahrbares, zur Einführung in die jeweilige Tubenöffnung bestimmtes Zentrierelement vorgesehen sein. Dieses Zentrierelement wird nach erfolgtem Einstechvorgang wieder in das Führungsrohr zurückgezogen.

Besonders vorteilhaft ist es, den sogenannten Funktionskopf, welcher die Elektroden und die Mittel zur Klemmkrafterzeugung umfaßt, als mechanisch und elektrisch kuppelbare Steckeinheit auszubilden. Auf diese Weise werden Sterilisationsvorgänge vereinfacht, und es kann sogar aufgrund der Einfachheit derartiger Funktionsköpfe vorgesehen sein, diese Funktionsköpfe als gegebenenfalls sterilisiert zugelieferte Einwegprodukte auszugestalten.

Ausführungsbeispiele der Erfindung werden nachfolgend unter Bezugnahme auf die Zeichnung erläutert; in dieser zeigt:
- Figur 1: eine schematische Darstellung einer ersten Ausführungsform der Vorrichtung nach der Erfindung mit mechanischer Klemmkrafterzeugung, und
- Figur 2: eine Prinzipdarstellung einer Schaltskizze für eine weitere Ausführungsvariante der Erfindung mit elektrischer Klemmkrafterzeugung.

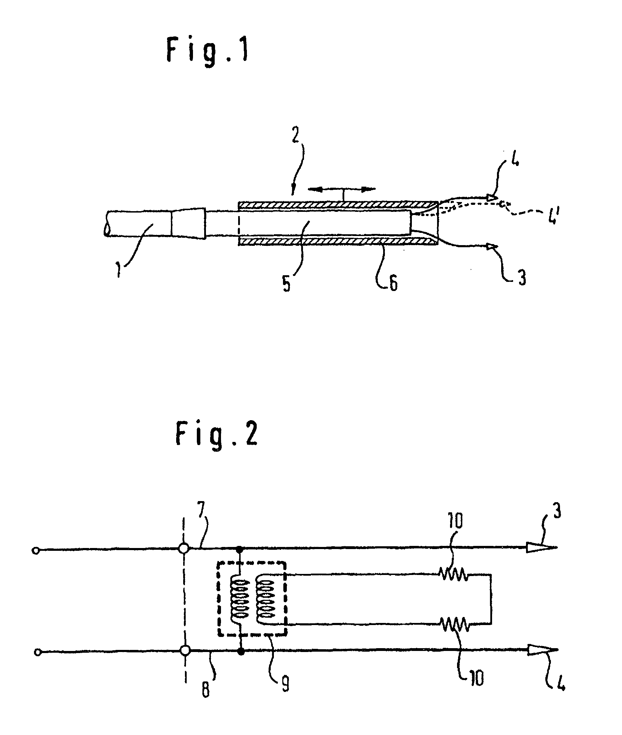

Nach Figur 1 umfaßt eine Vorrichtung nach der Erfindung ein in der Zeichnung nur teilweise dargestelltes Trägerrohr 1, das mit einem Funktionskopf 2 mechanisch und elektrisch kuppelbar ist. In dem Trägerrohr 1 bzw. dem Führungsrohr 5 des Funktionskopfs 2 sind zwei einander diametral gegenüberliegende Elektroden 3, 4 axial verschiebbar angebracht.

Diese Elektroden 3, 4 befinden sich im zurückgezogenen Zustand innerhalb des Führungsrohrs 5 und nehmen im ausgefahrenen Zustand die in Figur 1 mit voll ausgezeichneten Linien dargestellte Position ein, in der sie einen definierten gegenseitigen Abstand aufweisen.

Diese Elektroden 3, 4 sind als feine, mit einer Spitze und / oder mit Schneidkanten versehene Metallelektroden ausgebildet.

Um zwischen den Elektroden 3, 4 eine Klemmkraft nach Art einer Klemmzange wirksam werden zu lassen, ist bei dem dargestellten Ausführungsbeispiel eine verschiebbare Überwurfhülse 6 auf dem Führungsrohr 5 angebracht, die vom außenliegenden Ende des Trägerrohrs her betätigbar und über das vordere Ende des Führungsrohrs verschiebbar ist. Dabei trifft die Hülse 6 auf die sich in einer Spreizstellung befindenden Elektroden 3, 4 und überführt sie in die strichliert dargestellte Arbeitsstellung, in der über die Elektroden 3, 4 ein Koagulationsstrom geleitet wird.

Die Überführung der Elektroden 3, 4 von der Ruhestellung in die Arbeitsstellung erfolgt nach dem Einstechen der Elektroden und deren Positionierung beiderseits des Eileiterkanals, so daß bei diesem Übergang von der Ruhestellung in die Arbeitsstellung ein zangenartig wirkender Druck auf das Gewebe ausgeübt und damit das Ostium komprimiert wird. Erfolgt in diesem Zustand, das heißt in der Arbeitsstellung ein Zusehalten des Koagulationsstroms, dann wird durch die sich einstellende Koagulation der jeweilige Eileiter dauerhaft verlötet bzw. verschlossen. Der Funktionskopf 2 wird bevorzugt als selbständige Einheit ausgebildet, die mit dem Trägerrohr 1 mechanisch und elektrisch kuppelbar ist. Bei dieser Kupplung müssen natürlich die erforderlichen elektrischen Anschlüsse hergestellt werden, das heißt es sind geeignete Steckverbindungen vorgesehen.

Die Schaltskizze nach Figur 2 erläutert die Funktionsweise einer mit elektrischer Klemmkrafterzeugung realisierten Vorrichtung nach der Erfindung. Die Elektroden 3, 4 sind in der Schaltskizze nur schematisch angedeutet, sie können hinsichtlich ihrer Formgebung den Elektroden nach Figur 1 entsprechen, aber die Besonderheit besteht nach dieser Ausführungsform darin, daß diese Elektroden 3, 4 mit Bimetallelementen kombiniert sind oder bevorzugt aus einem Bimetall bestehen.

Durch Verwendung wenigstens einer Heizwendel und vorzugsweise durch Zuordnung jeweils einer Heizwendel zu einer Bimetallspitze wird erreicht, daß bei Beaufschlagung der Heizwendel mit Strom die Bimetallspitzen erwärmt werden und sich damit in definierter Weise verformen und aufeinander zu bewegen, das heißt nach Art einer gelenkfreien Zange wirken.

Der für die Heizwendel 10 benötigte Heizstrom wird vorzugsweise vom Koagulationsstrom über einen Transformator 9 abgezweigt. Der hochfrequente Teilstrom wirkt in den Heizwendeln 5 praktisch wie ein Gleichstrom und ist damit als Heizstrom bestens geeignet.

Durch die Verwendung eines vom Koagulationsstrom abgezweigten Heizstroms wird der Vorteil erreicht, daß durch einfache Bedienung des Schalters für den Koagulationsstrom gleichzeitig die Klemmkrafterzeugung ausgelöst bzw. bewirkt wird, was eine besonders einfache Ausgestaltung der Vorrichtung ermöglicht.

Wird der Funktionskopf der Vorrichtung nach der Erfindung als selbständige Einheit ausgebildet, dann wird der Transformator 9 vorzugsweise vor der Koppelstelle im Führungs- oder Trägerrohr angeordnet, und es müssen am Funktionskopf vier Steckverbindungen zur Herstellung der elektrischen Anschlüsse vorgesehen werden. Dies bereitet jedoch in der praktischen Realisierung in Verbindung mit einer geeigneten mechanischen Kupplung keine Schwierigkeiten.

Bei Verwendung von als Stech- und Klemmelemente ausgebildeten Elektroden aus Bimetall oder unter Verwendung von Bimetall sind diese Elektroden bevorzugt unverschieblich angebracht und mit dem Funktionskopf fest verbunden, so daß keinerlei Schiebemechanik, insbesondere auch keine Mechanik zum Ausschieben dieser Elektrode aus einem Führungsrohr erforderlich ist.

Gegebenenfalls kann im Führungsrohr noch mittig zwischen den Elektroden ein verfahrbares, zur Einführung in die jeweilige Tubenöffnung bestimmtes Zentrierelement vorgesehen werden, da auf diese Weise eine besonders genaue Positionierung der Elektroden vor der Durchführung des Einstechvorgangs relativ zur Tubenöffnung möglich ist.

## Patentansprüche

1. Vorrichtung zum Verschließen von in den Uterus führenden Tuben mit einem bipolaren, mit einer hochfrequenten Stromquelle verbindbaren Koagulationssystem, wobei am Ende einer rohrförmigen Träger- und/oder Inspektionseinheit (1) ein Funktionskopf (2) mit zumindest zwei in Form von Klemmelementen ausgebildeten Elektroden (3, 4) vorgesehen ist, die zwischen einer passiven Position und einer aktiven Koagulationsposition verstellbar sind,
**dadurch gekennzeichnet,**
**dass** die Träger- und/oder Inspektionseinheit (1) mit dem Funktionskopf (2) als in den Uterus einführbare Einheit ausgebildet ist und die Enden der Elektroden (3, 4) die Form von Stechspitzen besitzen, die in der passiven Position einen ein Einstechen beiderseits der Eileiteröffnung gewährleistenden gegenseitigen Abstand besitzen, und dass dieser Abstand in der aktiven Koagulationsposition durch eine zur Wirkung kommende Klemmkraft verringert ist.

2. Vorrichtung nach Anspruch 1,
**dadurch gekennzeichnet,**
**dass** die Elektrodenenden Schneidkanten aufweisen.

3. Vorrichtung nach Anspruch 1 oder 2,
**dadurch gekennzeichnet,**
**dass** die Elektroden (3, 4) in einem Führungsrohr (5) angeordnet und aus diesem Führungsrohr (5) ausfahrbar sind, wobei der gegenseitige Abstand der Elektroden (3, 4) im Führungsrohr (5) geringer als im ausgefahrenen, der Stechposition entsprechenden Zustand ist.

4. Vorrichtung nach einem der vorhergehenden Ansprüche,
**dadurch gekennzeichnet,**
**dass** zur Überführung der Elektroden (3, 4) von der Stechposition in die Klemm- und Koagulationsposition eine mechanische und/oder elektrische Anordnung zur Klemmkrafterzeugung vorgesehen ist und die Einrichtung zur mechanischen Klemmkrafterzeugung ein relativ zum Führungsrohr (5) verschiebbares, an den Elektroden (3, 4) angreifendes und deren gegenseitigen Abstand verringerndes Betätigungsorgan (6) umfasst, das aus einem gesteuert auf dem Führungsrohr (5) verschiebbaren, außenseitig an den Elektroden (3, 4) angreifenden Hülsenelement besteht.

5. Vorrichtung nach Anspruch 4,
**dadurch gekennzeichnet,**
**dass** die elektrische Anordnung zur Klemmkrafterzeugung mit den Elektroden (3, 4) verbundene Bimetallelemente umfasst, die über zugeordnete Heizwendeln (10) aufheizbar sind.

6. Vorrichtung nach Anspruch 5,
. **dadurch gekennzeichnet,**
**dass** die Elektroden (3, 4) selbst in Form von Bimetallelementen ausgebildet sind.

7. Vorrichtung nach Anspruch 5 oder 6,
**dadurch gekennzeichnet,**
**dass** die Elektroden (3, 4) am Funktionskopf (2) unverschieblich und sich in der Einstechposition befindend angebracht sind.

8. Vorrichtung nach Anspruch 5,
**dadurch gekennzeichnet,**
**dass** im Trägerelement (1) mit Abstand vom Funktionskopf (2) ein Transformator (9) zwischen Koagultaionsstrom-Zuführleitungen (7, 8) angeordnet ist und dass mittels des Transformators (9) ein Teilstrom zur Speisung der Heizwendeln (10) abgezweigt ist.

9. Vorrichtung nach Anspruch 8,
**dadurch gekennzeichnet,**
**dass** ein Schalter zur zeitgleichen Zuschaltung von Koagulations- und Heizwendelstrom vorgesehen ist.

10. Vorrichtung nach Anspruch 8,
**dadurch gekennzeichnet,**
**dass** eine elektrisches Verzögerungsglied vorgesehen ist, das die Zuschaltung des Koagulationsstroms bezüglich der Zuschaltung des Heizwendelstroms zeitlich verzögert.

11. Vorrichtung einem der vorhergehenden Ansprüche,
**dadurch gekennzeichnet,**
**dass** der Funktionskopf (2) als mit der Trägereinheit (1) mechanisch und elektrisch kuppelbare Steckeinheit ausgebildet ist.

12. Vorrichtung nach Anspruch 3,
**dadurch gekennzeichnet,**
**dass** im Führungsrohr (5) ein mittig zwischen den Elektroden (3, 4) verfahrbares, zur Einführung in die jeweilige Tubenöffnung bestimmtes Zentrierelement vorgesehen ist.

## Claims

1. An apparatus for the closing of tubes leading into the uterus having a bipolar coagulation system which can be connected to a radiofrequency current source, with a functional head (2) being provided at the end of a tubular carrier and/or inspection unit (1) and having at least two electrodes (3, 4) in the form of clamping elements which can be adjusted between a passive position and an active coagulation position,
**characterised in that** the carrier and/or inspection unit (1) is formed as a unit insertable into the uterus with the functional head (2) and **in that** the ends of the electrodes (3, 4) have the form of lance tips which have a mutual spacing in the passive position which ensures a lancing at both sides of the opening of the fallopian tube; and **in that** this spacing is reduced in the active coagulation position by a clamping force which comes into action.

2. An apparatus in accordance with claim 1, **characterised in that** the electrode ends have cutting edges.

3. An apparatus in accordance with claim 1 or claim 2, **characterised in that** the electrodes (3, 4) are arranged in a guide tube (5) and can be extended out of this guide tube (5), with the mutual spacing of the electrodes (3, 4) in the guide tube (5) being smaller than in the extended state corresponding to the lancing position.

4. An apparatus in accordance with any one of the preceding claims,
**characterised in that** a mechanical and/or electrical arrangement is provided for the production of the clamping force for the moving of the electrodes (3, 4) from the lancing position into the clamping and coagulation position and **in that** the device for the mechanical production of the clamping force includes an actuation member (6) which is displaceable relative to the guide tube (5), acts at the electrodes (3, 4) and reduces their mutual spacing and consists of a sleeve element which is controllably displaceable on the guide tube (5) and acts on the electrodes (3, 4) at the outer side.

5. An apparatus in accordance with claim 4, **characterised in that** the electrical arrangement for the production of the clamping force includes bimetallic elements connected to the electrodes (3, 4) and heatable via associated heating spirals (10).

6. An apparatus in accordance with claim 5, **characterised in that** the electrodes 3, 4 are themselves made in the form of bimetallic elements.

7. An apparatus in accordance with claim 5 or claim 6, **characterised in that** the electrodes (3, 4) are attached immovably to the functional head (2) when located in the lancing position.

8. An apparatus in accordance with claim 5, **characterised in that** a transformer (9) is arranged between the coagulation current supply lines (7, 8) in the carrier element (1) and spaced apart from the functional head (2); and **in that** a part current is branched off by means of the transformer (9) for the feeding of the heating spirals (10).

9. An apparatus in accordance with claim 8, **characterised in that** a switch is provided for the simultaneous switching on of the coagulation current and the heating spiral current.

10. An apparatus in accordance with claim 8, **characterised in that** an electric delay member is provided which causes a time delay for the switching on of the coagulation current with respect to the switching on of the heating spiral current.

11. An apparatus in accordance with any one of the preceding claims, **characterised in that** the functional head (2) is formed as a plug connection unit which can be mechanically and electrically coupled to the carrier unit (1).

12. An apparatus in accordance with claim 3, **characterised in that** a centring element is provided which can be moved centrally between the electrodes (3, 4) and is intended for insertion into the respective tube opening.

## Revendications

1. Dispositif destiné à l'obturation de conduits aboutissant dans l'utérus, comportant un système de coagulation bipolaire pouvant être relié à une source de courant haute fréquence, une tête fonctionnelle (2) étant prévue au niveau de l'extrémité d'une unité de support et/ou d'inspection (1) tubulaire et étant munie d'au moins deux électrodes (3, 4) agencées sous la forme d'éléments à serrage, qui sont réglables entre une position passive et une position active de coagulation, **caractérisé en ce que**
l'unité de support et/ou d'inspection (1) avec la tête fonctionnelle (2) est agencée en tant qu'unité pouvant être introduite dans l'utérus, et **en ce que** les extrémités des électrodes (3, 4) ont la forme de pointes de piquage qui, dans la position passive, sont positionnées à un écartement mutuel assurant un piquage de part et d'autre de l'ouverture de la trompe, et **en ce que**, dans la position active de coagulation, cet écartement est réduit par l'effet d'une force de serrage.

2. Dispositif selon la revendication 1, **caractérisé en ce que**
les extrémités des électrodes comportent des arêtes coupantes.

3. Dispositif selon la revendication 1 ou 2, **caractérisé en ce que**
les électrodes (3, 4) sont disposées dans un tube de guidage (5), et peuvent être retirées de ce tube de guidage (5), l'écartement mutuel entre les électrodes (3, 4) dans le tube de guidage (5) étant inférieur à celui de l'état retiré correspondant à la position de piquage.

4. Dispositif selon l'une quelconque des revendications précédentes, **caractérisé en ce que**,
pour la transposition des électrodes (3, 4) de la position de piquage dans la position de serrage et de coagulation, il est prévu un système mécanique et/ou électrique destiné à générer une force de serrage, et **en ce que** le système destiné à générer une force de serrage mécanique comporte un organe d'actionnement (6) pouvant être déplacé par rapport au tube de guidage (5), qui agit sur les électrodes (3, 4) et réduit leur écartement mutuel, lequel est constitué d'un élément de douille commandé pouvant être déplacé sur le tube de guidage (5), et agissant sur la face extérieure des électrodes (3, 4).

5. Dispositif selon la revendication 4, **caractérisé en ce que**
le système électrique destiné à générer une force de serrage comporte des éléments bimétalliques reliés aux électrodes (3, 4), qui peuvent être chauffés par des filaments de chauffage (10) associés.

6. Dispositif selon la revendication 5, **caractérisé en ce que**
les électrodes (3, 4) sont elles-mêmes réalisées sous la forme d'éléments bimétalliques.

7. Dispositif selon la revendication 5 ou 6, **caractérisé en ce que**
les électrodes (3, 4) sont fixées sur la tête fonctionnelle (2) de façon à ne pas pouvoir se déplacer, et se trouvent dans la position de piquage.

8. Dispositif selon la revendication 5,
**caractérisé en ce que**
un transformateur (9) est disposé dans l'élément de support (1) à un certain écartement de la tête fonctionnelle (2) entre des lignes d'amenée de courant de coagulation (7, 8), et **en ce qu'**un courant partiel pour l'alimentation des filaments de chauffage (10) est dérivé au moyen du transformateur (9).

9. Dispositif selon la revendication 8, **caractérisé en ce que**,
il est prévu un commutateur pour la mise en circuit simultanée du courant de coagulation et du courant des filaments de chauffage.

10. Dispositif selon la revendication 8, **caractérisé en ce que**,
il est prévu un élément de temporisation électrique, qui retarde la mise en circuit du courant de coagulation par rapport à la mise en circuit du courant des filaments de chauffage.

11. Dispositif selon l'une quelconque des revendications précédentes, **caractérisé en ce que**
la tête fonctionnelle (2) est réalisée sous la forme d'une unité enfichable pouvant être couplée mécaniquement et électriquement à l'unité de support (1).

12. Dispositif selon la revendication 3, **caractérisé en ce que**
dans le tube de guidage (5) un élément de centrage est prévu et peut être déplacé au centre entre les électrodes (3, 4) pour l'insertion dans l'ouverture de conduit concerné.
